# EUROPEAN PATENT SPECIFICATION

(11) **EP 4 189 375 B1**
(45) Date of publication and mention of the grant of the patent: **28.01.2026**
(21) Application number: 21746501.2
(22) Date of filing: 29.07.2021
(51) Int. Cl.: B01L 9/00, G01N 27/27, B01L 3/00, G01N 33/49, G01N 33/543

(54) **BIOSENSOR SYSTEM FOR MULTIPLEXED DETECTION OF BIOMARKERS**
BIOSENSORSYSTEM ZUR MULTIPLEXEN DETEKTION VON BIOMARKERN
SYSTÈME DE BIOCAPTEUR POUR LA DÉTECTION DE BIOMARQUEURS MULTIPLEXÉE

(30) Priority: 03.08.2020 EP 20382721
(43) Date of publication of application: 07.06.2023
(73) Proprietor: Consejo Superior de Investigaciones Científicas (CSIC), 28006 Madrid (ES)
(72) Inventor: FERNÁNDEZ SÁNCHEZ, César, 28006 Madrid (ES); BALDI COLL, Antoni, 28006 Madrid (ES); GUTIÉRREZ CAPITÁN, Manuel, 28006 Madrid (ES)
(74) Representative: Hoffmann Eitle
(86) International application number: PCT/EP2021/071324
(87) International publication number: WO 2022/029013

(56) References cited:
- EP-A1- 1 223 425
- EP-A1- 2 515 103
- EP-A1- 3 335 794
- US-A- 5 520 787
- US-A1- 2004 106 190
- US-A1- 2011 042 237
- US-A1- 2011 286 888
- US-A1- 2015 041 337
- US-A1- 2017 138 934
- PABLO GIMÉNEZ-GÓMEZ ET AL: "Microfluidic Modules with Integrated Solid-State Sensors for Reconfigurable Miniaturized Analysis Systems", ACS OMEGA, vol. 4, no. 4, 3 April 2019 (2019-04-03), US, pages 6192 - 6198, XP055763478, ISSN: 2470-1343, DOI: 10.1021/acsomega.9b00064
- ELSON LUIZ FAVA ET AL: "Electrochemical paper-based microfluidic device for high throughput multiplexed analysis", TALANTA, vol. 203, 1 October 2019 (2019-10-01), NL, pages 280 - 286, XP055763857, ISSN: 0039-9140, DOI: 10.1016/j.talanta.2019.05.081

## Description

### Field and object of the invention

The present invention refers in general to biosensor systems for quick and multiplexed detection of biomarkers present in biological fluids.

An object of the invention is to provide biosensor systems of reduced size, and that additionally reduce analysis costs and material waste.

An additional object of the invention is to provide a biosensor system that simplifies scalability of an array of electrochemical cells to be applied, for example for ELISA assays.

An additional object of the invention if to provide a multiplexed biosensor system that combines a reusable array of electrochemical cells and a disposable microfluidic component.

### Background of the invention

There are many different types of biosensors to electrochemically analyze biomarkers contained in biological fluids like: blood, serum, sweat, tear, urine, saliva, sputum, nasopharyngeal and oropharyngeal specimens. A biomarker is a substance used as an indicator of a normal biological process, disease or response to a drug treatment.

Measuring enzyme activity for a specific substance contained in a biofluid with good reproducibility, is important for an electrochemical biosensor based on enzymatic detection approach such as, for example, glucose sensor, uric acid sensor, protein sensor, or a DNA sensor for clinical chemical tests.

Some known biosensors combine a fluidic component in paper (cellulose or nitrocellulose) and a matrix or array of miniaturized electrochemical cells, that are usually manufactured using screen-printing An example thereof is described by Elson Luiz Fava et al in "Electrochemical paper-based microfluidic device for high throughput multiplexed analysis", TALANTA, vol. 203, pages 280-286.

Typically, both, the fluidic component and the array of transducers are designed as disposable components, such that after a test has been performed, both components are discarded. Therefore, these type of clinical analysis or tests, generate a significant amount of waste.

Furthermore, one of the most important limitations when designing compact electrochemical cells in a planar configuration is the space on the substrate occupied by electrical tracks and associated contact pads of electrodes operating independently. For a conventional three-electrode electrochemical cell configuration, (working, counter and reference electrodes WE, CE, and RE), the number of required connections when fabricating electrochemical cell arrays is three times the number of cells, which greatly limit the size of the array that can be defined on one substrate, and increase manufacturing complexity and cost.

Different approaches to overcome this limitation have been proposed, generally based on the use of grid-patterned electrodes inserted in specially designed microfluidic devices to enabling multiplex detection, or using a travelling light pointer to form transient working electrodes or sharing working electrode connections and multiple fluid compartments. Even though a high density of working electrodes is achieved, external or on-chip counter electrodes and/or reference electrodes are shared among many working electrodes immersed in the same liquid pool.

This hampers the use of fully addressable electrochemical cells that could be in contact with solutions of different composition, as required for example for electrochemical ELISA assays carried out in conventional 96-well microtiter plates.

### Summary of the invention

The present invention is defined in the attached independent claim, and satisfactorily solves the shortcomings of the prior art, by providing a biosensor system of reduced size for the multiplexed and quick detection of biomarkers in biological samples like for example: blood, serum, sweat, tear, urine, saliva, sputum, nasopharyngeal and oropharyngeal specimens. In the case of non-fluid biological samples, these are pre-treated in a conventional manner to obtain fluid form of the same for the biomarkers detection.

The biosensor system of the invention comprises: a reusable array comprising at least two individual electrochemical cells that are individually addressable, a disposable fluidic component, and a cartridge to integrate and align the array and the microfluidic component.

The at least two individual electrochemical cells were characterized in isolated liquid wells patterned in a polymeric structure, and arranged in correspondence with the electrochemical cells for the electrochemical detection of individual samples contained in each liquid well.

The system further comprises a set of working electrodes, a counter electrode and a reference electrode common to all the electrochemical cells, such that each electrochemical cell includes one working electrode and a defined area of the shared counter electrode and reference electrode.

The biosensor system comprises two shared electrodes, namely a counter electrode and reference electrode, such that the counter and reference electrodes are common electrodes for all the working electrodes, that is, the counter and reference electrodes are shared by all the electrochemical cells (three-electrode configuration).

Preferably, the counter electrode and the reference electrode are straight tracks parallel to each other, and parallel to the longitudinal alignment direction of the working electrodes. Furthermore, the longitudinal alignment direction of the working electrodes, is placed in between the counter electrode and the reference electrode.

Preferably, the liquid wells used to characterize electrochemical cells, are also aligned in a straight longitudinal direction, following the parallel arrangement of the counter and reference electrode and to the longitudinal alignment direction of the working electrodes.

The configuration of the electrochemical cells allows multi-parametric or multisample analysis, because a desired number of electrochemical cells can be included in a transducer chip design. The electrochemical cells are individually addressable, that is, a determination can be made in each electrochemical cell, either of the same biomarker in different samples, or different biomarkers in the same sample.

The system further comprises a set of connection pads, and a set of connection tracks connecting the working electrodes, the shared counter electrode and the shared reference electrode, with the connection pads. Preferably, a part of each connection track is parallel to the shared counter and shared reference electrodes.

The system comprises a disposable fluidic component, made of a porous material for driving fluid by capillary to the electrochemical cells. The disposable fluidic component is made of a cost-effective material, preferably paper (cellulose or nitrocellulose), and includes distributed fluidic channels defined and isolated by hydrophobic barriers. The fluidic component formed as a substrate, is operatively couplable with the array of electrochemical cells, for performing electrochemical detection.

Therefore, taking into account the above-described embodiment, the system of the invention is based on the combination of a fluidic component and a matrix or array of electrochemical cells, wherein the fluidic component is discarded after each analysis, and the electrochemical cell component is a reusable part of the device, so that, less waste is generated and the cost per analysis is reduced. This represents a difference with the usual electrochemical tests used in biological measurements, where both paper strip and electrochemical cell are disposable.

The system additionally comprises a cartridge having top and bottom parts couplable to each other, where a chip substrate is enclosed comprising the connection pads and the electrodes to configure the array of electrochemical cells.

Preferably, the chip substrate is generally rectangular and the connection pads are grouped in a reticular matrix arrangement and formed adjacent to one short side of the substrate. A set of spring-loaded connectors is provided for their connection with the electrode contact pads of the electrochemical cells.

The disposable fluidic component can be inserted in the cartridge and pressed between the two parts and operatively coupled with the array of electrochemical cell.

The fluidic channels have the form of strips, for example parallel to each other, such that when the disposable fluidic component is coupled with the electrochemical cells, the fluidic channels are transversally arranged with respect to the shared counter and reference electrodes and the working electrodes of each electrochemical cell.

### Brief description of the drawings

Preferred embodiments of the invention are henceforth described with reference to the accompanying drawings, wherein:
Figure 1.- shows a top plan view of an array of electrochemical cells not according to the invention, that includes a layout of a two-electrode configuration
Figure 2.- shows a top plan view of an implementation of the array of electrochemical cells, that includes a layout of three-electrode configuration.
Figure 3.- shows an exploded view of a polymeric cartridge defining liquids wells.,
Figure 4.- shows in a top plan view, a fluidic component (Figure A), and the arrangement of the fluidic component coupled with the array of electrochemical cells (Figure B) and an absorbent pad overlapping the channels of the fluidic component.
Figure 5.- shows in plan view the layout of the component once coated with vinyl layers for better alignment with the array of electrochemical cells. Figure A is a plan view of the upper side, Figure B is a plan view of the bottom side, and Figure C is a top plan view of the component coupled with the array of electrochemical cells.
Figure 6.- shows in Figure A a top plan view of the layout of a device packaging to easily couple and align the fluidic component with the array of electrochemical cells, and in Figure B an exploded view of the packaging components including the clamping structures used por applying a constant pressure between the top and bottom parts of the cartridge.
Figure 7.- shows a graph of a cyclic voltammogram recorded with one electrochemical cell in a 0.1 M TRIS. HCI buffer solution pH 9.8 containing 1 mM BQ electroactive species. Scan rate= 50 mV/s.
Figure 8.- shows in Figures 8A - 8C graphs of the calibration curves for the three biomarkers with ferrocene-methanol as redox mediator.
Figure 9.- shows the results of the measurement of the three biomarkers in human sputum samples.

### Preferred embodiment of the invention

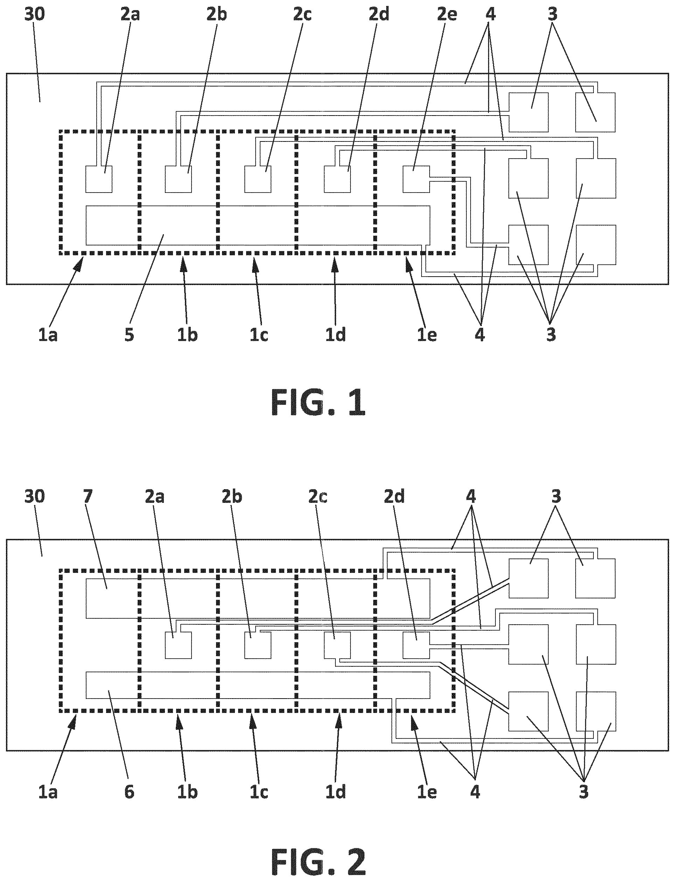

**Figure 1** shows an implementation (not according to the present invention) of the layout of the electrodes, connection pads (3) and connection tracks formed on a surface of a substrate (30) to produce an array of electrochemical cells (1a,1b,1c,1d,1e) in a two-electrode configuration. A plurality of working electrodes (2a,2b,2c,2d,2e) are aligned along a straight longitudinal direction, and one counter/reference electrode (5) is provided common for all the working electrodes (2a,2b,2c,2d,2e). The counter/reference electrode (5) is a straight track and the working electrodes are arranged adjacent to one side of the shared counter/reference electrode (5), in a way that the longitudinal direction of alignment of the working electrodes, is parallel to the counter/reference electrode (5).

The substrate (30) is rectangular and includes a set of connection pads (3) that are placed adjacent to one short side of the substrate (30) as shown in **Figure 1****.** The connection pads (3) are grouped in a reticular matrix arrangement for their connection with a group of spring-loaded connectors, as it will be explained later on.

A plurality of the connection tracks (4) connect individually each working electrode (2a,2b,2c,2d,2e) and the counter/reference electrode (5), each with one connection pad (3). As represented in **Figure 1****,** the connection tracks run longitudinally in the substrate (30), in a way that a part of each connection track is parallel to the shared counter / reference electrode (5).

As it can be noted in **Figure 1****,** with this layout, where the counter/reference electrode is common to all the working electrodes, the number of connection tracks and connection pads, is significantly reduced so that the substrate (30) features a compact design.

Each electrochemical cell (1a,1b,1c,1d,1e) comprises one working electrode (2a,2b,2c,2d,2e) and the shared counter/reference electrode (5), thus, in the embodiment of **Figure 1** there are five electrochemical cells that could be integrated in a single device.

**Figure 2** shows an electrode layout on the substrate (30) for a three-electrode cell configuration. In this case, there are four working electrodes (2a,2b,2c,2d), a reference electrode (6), a counter electrode (7), a group of connection pads (3), and a plurality of connection tracks (4) connecting individually the working electrodes with the connection pads (3). The reference and the counter electrodes (6,7), are common for all the working electrodes, so they are shared by all the electrochemical cells (1a,1b,1c,1d,1e).

The working electrodes (2a,2b,2c,2d) are also aligned, and the reference and counter electrodes (6,7) are straight tracks parallel to each other, and parallel to the longitudinal alignment direction of the working electrodes. The working electrodes are arranged in between the reference and the counter electrodes (6,7). Several connection tracks (4) connect individually all the electrodes (2,6,7) with the connection pads, and have a part parallel to the reference and the counter electrodes (6,7).

In this case, each electrochemical cell (1a,1b,1c,1d,1e) includes a working electrode (2a,2b,2c,2d) and a defined area of the reference and counter electrodes (6,7).

The embodiment of **Figure 2** includes four electrochemical cells (1b,1c,1d,1e) in a three-electrode configuration for the simultaneous measurement of biomarkers, and an additional cell (1a) including only two electrodes (the counter and the reference electrodes), which is used as reference channel.

In both implementations described above, all the electrodes of the electrochemical cells can be manufactured by standard photolithographic techniques, by patterning gold or platinum electrodes on silicon substrates.

As it can be observed in **Figures 1** and **2****,** the fact that all electrochemical cells share counter/reference electrode (two-electrode cells) or a common counter and reference electrodes (three-electrode cells), has the advantage that only six contact pads are required, which greatly reduce the number of contact pads for connecting externally all the cells.

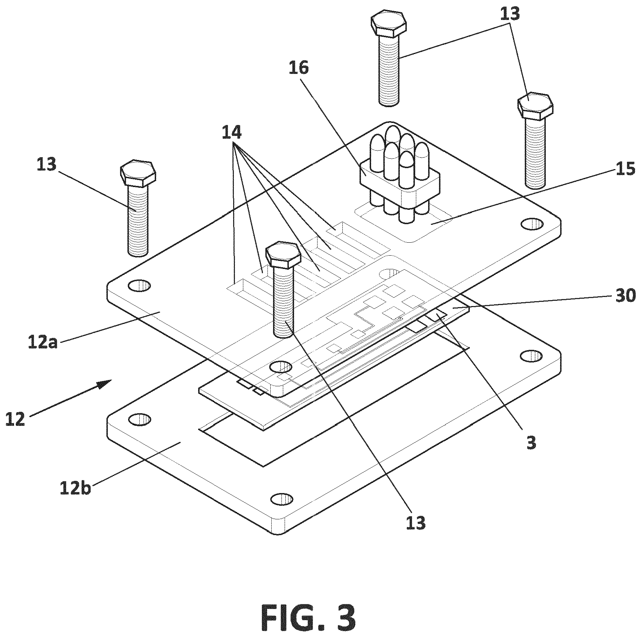

As represented in **Figure 3****,** the array of electrochemical cells (1a,1b,1c,1d,1e) can be individually characterized in a cartridge (12) having top and bottom parts (12a,12b) couplable to each other, for example by means of screws (13) or other type of clamping means, so that the array is enclosed between the top and bottom parts (12a,12b). The top part (12a) is a substrate having several windows that configure isolated liquid wells (14), that are distributed such that each well (14) is placed right on top of an electrochemical cell.

In a practical implementation of the invention, the capacity of each well (14) is around 75-µL.

In the preferred embodiment represented in the **Figure 3****,** the wells (14) are aligned in a straight longitudinal direction, that is parallel to the shared reference and counter electrodes, and parallel to the longitudinal alignment direction of the working electrodes.

The top part (12a) has an additional window (15) for the insertion of a set of spring-loaded connectors (16), located right on the connection pads (3), in a way that when the cartridge is assembled, each connector (16) would be connected to a connection pad (3). The connectors (16) serve to connect the electrochemical cells with an external measurement device for the electrochemical cell characterization.

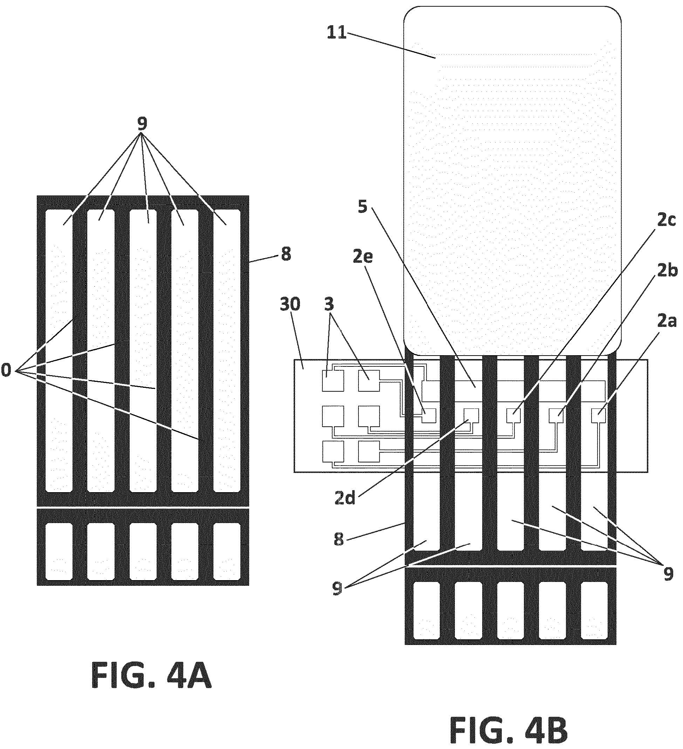

As shown in **Figure 4A****,** the biosensor system includes a disposable paper component (8) in the form of a substrate being a reactive fluidic component comprising fluidic channels (9) arranged in a way that the channels (9) are isolated by hydrophobic barriers (10), that can be realized as printed hydrophobic walls.

In this preferred embodiment, the fluidic channels (9) have the form of straight strips. However, in other preferred embodiment, the fluidic channels (9) might have other configurations suitable for each application.

The two implementations of **Figure 1** and **2** are designed such that they could be easily coupled to the paper microfluidic component (8). In addition, the design in both implementations features a reduced size as the overall dimensions of the device are 25x8 mm².

As represented in **Figure 4B****,** the paper component (8) is operatively couplable with the array of electrochemical cells (1a,1b,1c,1d,1e) formed on the substrate (30) for the electrochemical detection, such that, each channel (9) is aligned over an electrochemical cell (1a,1b,1c,1d,1e), that is, on a working electrode (2a,2b,2c,2d,2e) and on a part of the counter / reference electrode (5).

When the disposable paper component (8) is coupled with the electrochemical cell array, the fluidic channels (9) are transversally arranged, preferably orthogonally, with respect to the counter / reference electrode (5), and with respect to the alignment of the working electrodes (2a,2b,2c,2d,2e).

The system includes an absorbent pad (11) overlapped and in contact with the channels (9) of the paper component, for increasing capillary force in the disposable paper component (8) while driving the fluid towards the electrochemical cells (1a,1b,1c,1d,1e).

Having a very lost-cost easy-to-manufacture disposable paper component (8), reduces cost per analysis required for Point-Of-Care devices.

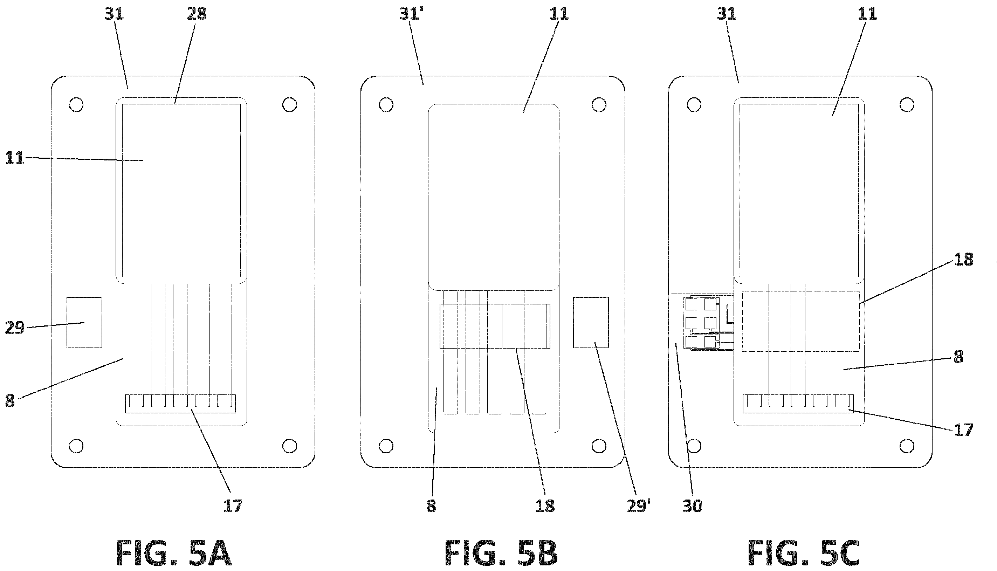

**Figure 5** shows the layout of the paper component (8) sandwiched between vinyl layers (31,31') for its easy alignment with the array of electrochemical cells. The vinyl layers (31,31') are patterned using a blade plotter, so that several areas of the paper component (8) are exposed.

As shown in **Figure 5A** a first window (17) is opened at a top surface for receiving samples, that leaves open the initial part of the paper channels for the sample addition. A second window (28) is opened at the sink area to allow liquid evaporation once it has reached the absorbent pad (11). A third window (18) is opened at the back side of the paper component (8) to provide access to the sensing areas of the same **(****Figure 5B****),** so that the paper component and the electrochemical cells can get in contact.

On both vinyl layers (31,31'), additional windows (29,29') are opened for the connector of the electrode array to be placed over the electrode contact pads. **Figure 5C** shows the relative positions of all the above-described windows.

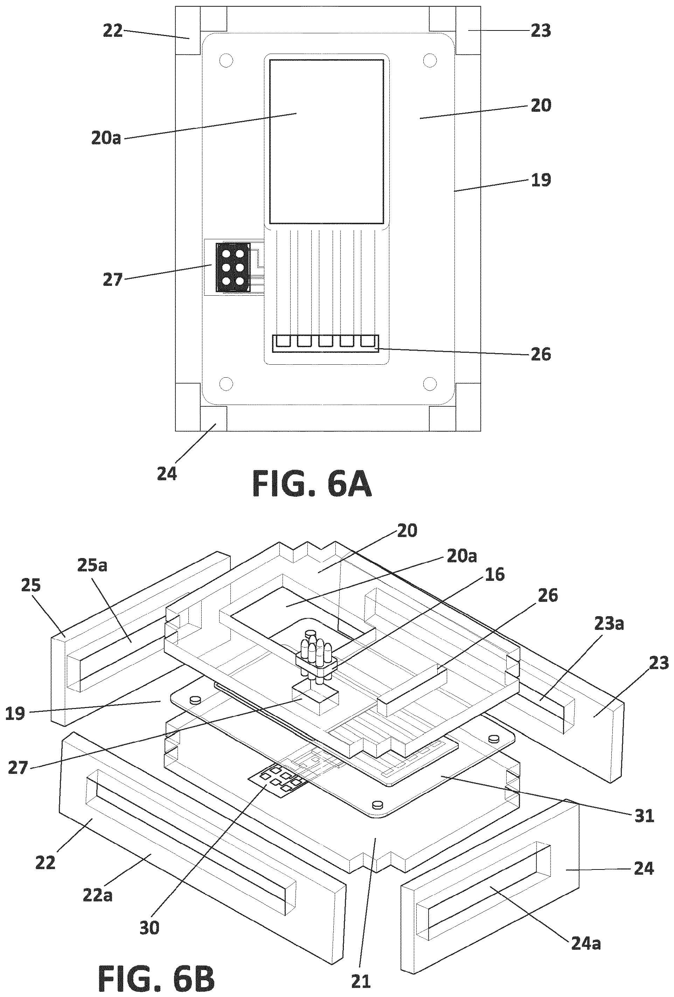

In practice, the coupling of the array of electrochemical cells and the disposable paper fluidic component (8) is done by means of a packaging cartridge (19) including top and bottom parts (20.21) and pressed together with clamping means, as shown in **Figure 6****.**

**Figure 6** illustrates the packaging cartridge (19) that integrates the packaged paper fluidic component (8) and the array of electrochemical cells (1a,1b,1c,1d,1e). The packaging cartridge (19) comprises top and bottom parts (20,21), and clamping means configured in this example as first and second large lateral walls (22,23), and first and second short lateral walls (24,25), such that, the lateral walls retain top and bottom parts (20,21) pressed against each other and keep both, the paper component and electrochemical cell array, robustly aligned during measurements.

The top part (20) has several windows to provide access to the packaging cartridge (19) interior, in particular it has: an evaporation window (20a) that is placed over the absorbent pad (11), and serves as sink area evaporation window, a sample access window (26) that is placed right over the first window (17) of the paper component (8), and a connector window (27) for receiving the spring-loaded connectors (16).

The bottom part (21) can integrate five 2-mm diameter Nd magnets (not shown) located right before the detection area of the electrode array, and aligned with the fluidic channels (9) so that, when working with magnetic nanoparticles, these flow on the paper and can be trapped and accumulated before carrying out the measurements.

As shown specially in **Figure 6B****,** top and bottom parts (20,21) are flat and generally rectangular and have cut-out corners, that conform protruding four edges in both covers.

Clamping means are provided for package assembly, wherein the clamping means are adapted to attach and press together top and bottom parts (20,21). In this exemplary embodiment, the clamping means are embodied as first and second large lateral walls (22,23) having respective windows (22a,23a), and first and second short lateral walls (24,25) have respective windows (24a,25a). The large lateral edges of the top and bottom covers (20,21) can be inserted respectively in the windows (22a,23a) of the large lateral walls, and the short edges of the top and bottom covers (20,21) can be inserted in the windows (24a,25a) of the two lateral short walls (24,25), conforming thereby a packaging cartridge (19) as the one shown in **Figure 6A****.**

Since the lateral walls are used as clamping means, the packaging cartridge (19) can be easily assembled and disassembled to get access to both the electrochemical cell array and the paper component so that the paper component can be easily removed after one measurement and replace it for a new one and the reusable array of electrochemical cells can be cleaned if necessary and replaced once its life cycle finishes. The packaging cartridge (19) can be manufactured at a low cost, because it does not require mechanical fastening means for its assembly. The package can be made for example by laser cutting of polymethylmethacrylate (PMMA) plates. Other polymeric materials can be used and patterned by injection molding or 3D printing.

In addition, the encapsulation of the paper component (8) with the vinyl layers (31,31') achieves a reproducible alignment between the electrochemical cells and the paper channel that does not rely on double-sided adhesives to bond the paper fluid component to the electrochemical cells as in prior art solutions. The packaging cartridge (19) applies an even pressure on the paper component (8), and ensures a proper contact between the paper channels and all the electrochemical cells in the array.

For the analytical assessment of the proposed electrochemical cell array, several experiments have been carried out with the three-electrode cell array in solutions containing ferrocyanide / ferricyanide redox pair by cyclic voltammetry. It was seen that the electrochemical response of each cell in the array was very similar to the one of an individual cell. Because of the cell configuration and in order to carry out the quasi-simultaneous measurements in all cells of the array, the two species of the redox pair should be present in solution. Working in this way, calibration curves for ferricyanide could be plotted for all cells in the array. The potential difference between the counter and reference electrode was kept very low (below 40 mV) during the timescale of the experiments. Thus, the use of a single counter/reference electrode was feasible and the two-electrode cell array was then characterised.

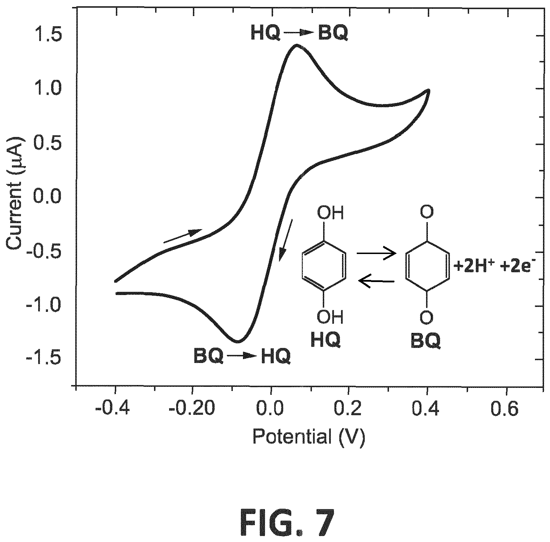

Similar experiments to the ones carried out with the previous array, have been performed and all the two-electrode cells in the array could be calibrated. As an example, the array was characterized by cyclic voltammetry (CV) in solutions containing ferrocyanide/ferricyanide or hydroquinone/benzoquinone (HQ/BQ) reversible redox pairs, as represented in **Figure 7****.** One of the species of the redox pair was measured with a linear response in individual cells of the array in the presence of an excess concentration of the redox counterpart.

Using the ferrocyanide / ferricyanide redox pair and as a first approach for measuring the activity of an enzyme label, some tests with Horseradish Peroxidase (HRP) have been carried out using ferrocyanide as the enzyme mediator. This enzyme converted ferrocyanide into ferricyanide product, which was measured by chronoamperometry using the two-electrode cell array. A similar experiment was performed with myeloperoxidase (MPO) but using ferrocene-methanol as a mediator as well as with alkaline phosphatase (ALP) using hydroquinone diphosphate (HQDP) as substrate.

In a practical application of the invention, a silicon chip of 8×25 mm2 is manufactured containing five electrochemical cells, with five working electrodes and a shared counter/pseudoreference electrode for all the cells. Since the array of electrochemical cells is reusable, the electrodes are made of gold.

The paper component of the Whatman Grade 1 Chromatographic type has 5 different channels. Together with the PMMA packaging, the determination of the concentration cytokine interleukin-8 (IL-8) and tumor necrosis factor alpha (TNF- α), together with the enzyme myeloperoxidase (MPO) in sputum samples has been addressed by a sandwich type magneto immunoassay. All three analytes are biomarkers of Chronic Obstructive Pulmonary Disease (COPD) and other inflammatory diseases of the respiratory system.

The electrochemical detection scheme of the device for the three biomarkers is as follows:
- the two-electrode cell array was implemented and the electrochemical transduction mode is chronoamperometry.
- MPO activity could be detected directly by using ferrocene-methanol redox substrate.
- HRP label together with ferrocene-methanol substrate was the alternative chosen for carrying out the electrochemical detection of TNF-α and IL-8.

Magnetic nanoparticles functionalized with primary antibodies are incubated with the sample, and after recognition, a second incubation is performed with a secondary antibody labeled with horseradish peroxidase (HRP) enzyme. The conjugated nanoparticles can then be concentrated, conditioned in a suitable solution and run through the paper component. A magnet embedded in the bottom part of the PMMA cartridge is capable of trapping them, and finally carrying out the electrochemical detection by adding a solution containing with the enzyme substrates to the paper component.

It was shown that both species could be easily detected by chronoamperometry and that just 5 µL of solutions were necessary for carrying out the measurements on each channel.

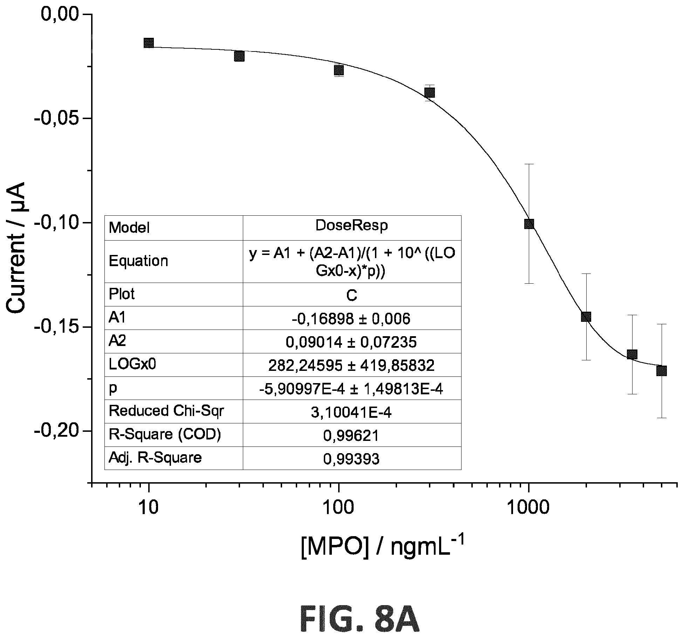

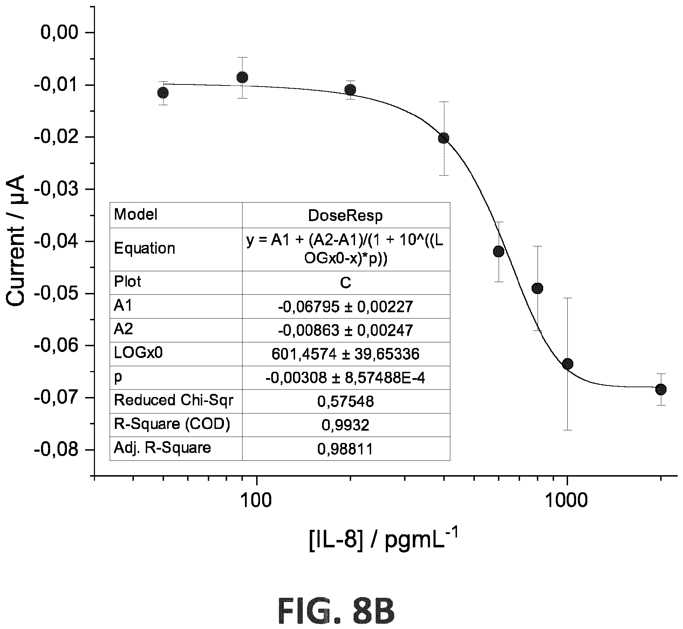

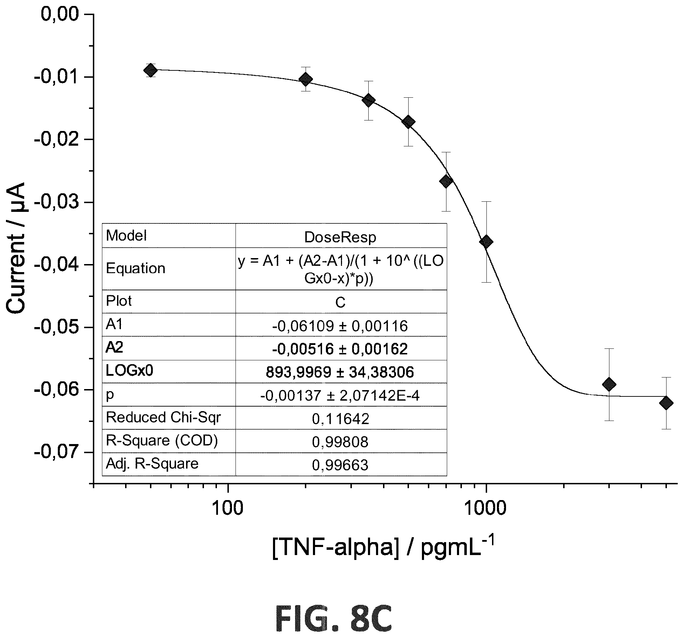

Then, measurements for the three biomarkers (TNF-α, IL-8 and MPO) were carried out in standard buffered solutions. These involved the following steps: Incubation of the antibody-coated magnetic nanoparticles in solutions containing different biomarker concentrations; washing; incubation of the magnetic nanoparticles in solutions containing the corresponding detection antibodies labelled with HRP for TNF- α and IL-8 assay; washing; casting of 5 µL of the MNP suspension on the paper channels; 2 rinsing steps, each of them with 5 µL rinsing solution; casting of 5 µL of enzyme substrate solutions and incubation for 3 min; final chronoamperometric measurement. The overall assay time is under 90 min. The calibration curves obtained for the three biomarkers in buffered solutions are represented in **Figure 8****.**

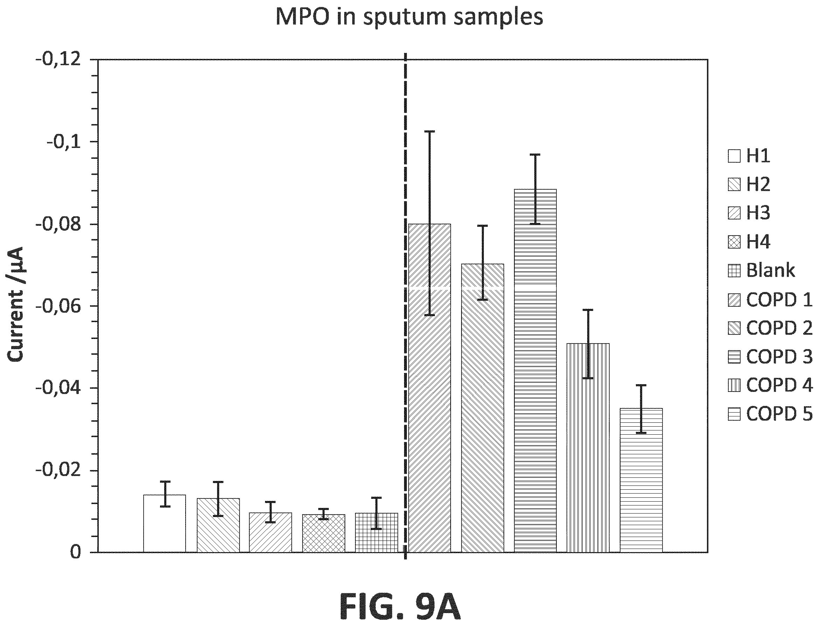

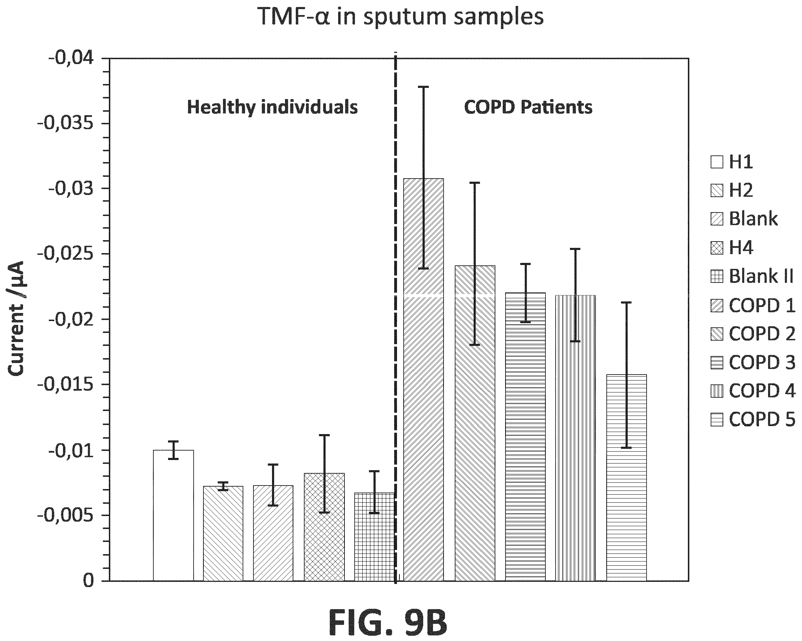

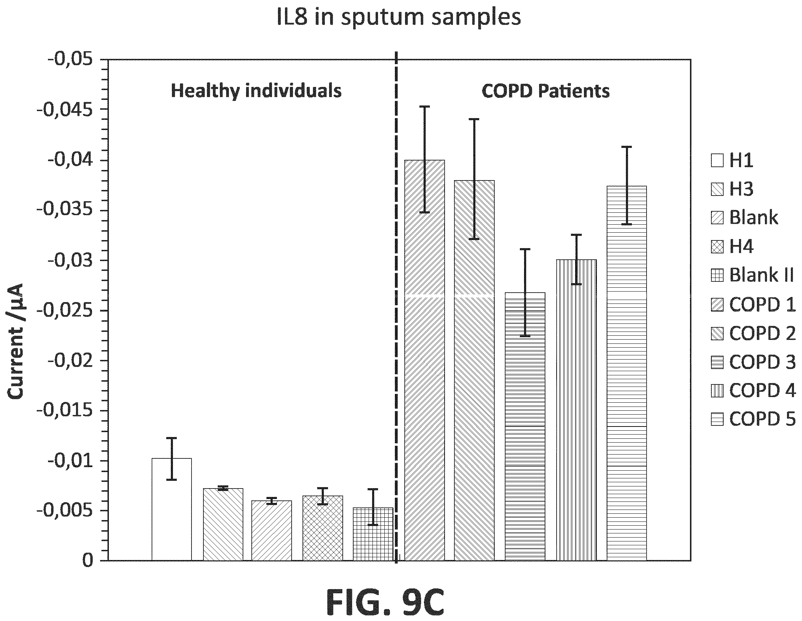

The same procedure was applied for the detection of MPO, TNF- α and IL-8 in human sputum samples. Five healthy individuals and five patients with COPD was used for the assay. The results shown a significant difference between the two groups of samples, as can be seen in **Figure 9****.**

This same procedure can be performed with different types of analytes, for example proteins, enzymes, viruses or bacteria, as well as DNA or RNA sequences, provided that we have the antibodies to perform the affinity assays or the necessary DNA strands.

According to the present invention, the two incubations are done directly in the device, running the functionalized magnetic nanoparticles on the paper while reacting with labelled antibodies or DNA strands previously deposited on the paper fluidic channels.

The biosensor system of the invention, can be marketed as a point-of-care or point-of-need device.

Other preferred embodiments of the present invention are described in the appended dependent claims and the multiple combinations of those claims.

## Claims

1. - A biosensor system for multiplexed detection of biomarkers, the system comprising:
an array of at least two electrochemical cells (1a,1b,1c,1d,1e), adapted to be individually addressable,
a working electrode (2a,2b,2c,2d,2e) in each electrochemical cell (1a, 1b, 1c, 1d, 1e) of the array,
a fluidic component (8) made of a porous material for driving fluids by capillary action towards the electrochemical cells (1a,1b,1c,1d,1e),
a cartridge (19) configured to align and put in contact the array and the fluidic component (8) for the electrochemical detection of biological samples;
a shared counter electrode (6) and a shared reference electrode (7), and
a chip substrate (30) enclosed inside the cartridge (19),
wherein the fluidic component (8) is made of paper having microfluidic channels (9) including biocomponents, wherein the microfluidic channels (9) are isolated from each other, wherein the fluidic component (8) is operatively couplable with the array of electrochemical cells (1a,1b,1c,1d,1e) such that each microfluidic channel (9) is placed over an electrochemical cell to carry out the electrochemical detection;
wherein the shared counter and reference electrodes (6,7) are common electrodes for all the electrochemical cells (1a, 1b,1c, 1d, 1e) of the array;
wherein the counter (6), reference (7), and working electrodes (2a,2b,2c,2d,2e) are formed on the same emeside of the chip substrate (30); and
wherein the microfluidic channels (9) are provided with antibodies or DNA strands to carry out affinity assays.

2. System according to claim 1, wherein the shared counter electrode (6) and the shared reference electrode (7) are straight tracks parallel to each other, and parallel to a longitudinal alignment direction of the working electrodes (2a,2b,2c,2d,2e), and wherein the working electrodes (2a,2b,2c,2d,2e) are aligned and arranged in between the shared counter electrode and a shared reference electrode.

3. System according to any of the preceding claims, further comprising a set of connection pads (3), and a set of connection tracks (4) connecting the working electrodes (2a,2b,2c,2d,2e), shared counter electrode and the shared reference electrode (6,7), with the connection pads (3), wherein a part of each connection tracks (4) is parallel to the shared counter and shared reference electrodes.

4. System according to claim 1, wherein the chip substrate (30) is rectangular and the connection pads (3) are grouped in a reticular matrix arrangement and formed adjacent to one short side of the substrate, and wherein the system further comprises spring-loaded contacts (16) connected with the connection pads (3).

5. System according to any of the claims 1 to 4, wherein the microfluidic channels (9) have the form of straight strips, such that when the fluidic component (8) is coupled with the electrochemical cells (1a,1b,1c,1d,1e), the microfluid channels (9) are transversally arranged with respect to the shared counter and reference electrodes (6,7).

6. System according to any of the preceding claims, wherein the cartridge (19) has top and bottom parts (20,21) and clamping structures (22,23,24,25) adapted to attach and press the top and bottom parts (20,21) of the cartridge (19) to keep the array and the fluidic component in close and stable contact.

7. System according to any of the claims 1 to 6, further comprising an absorbent pad (11) in contact and partially overlapping the microfluidic channels (9) of the fluidic component (8).

8. System according to claim 7, wherein the top part (20) of the cartridge (19) has an evaporation window (20a) that is placed over the absorbent pad (11), and serves as sink area evaporation window, and a sample access window (26) that is placed right over a first window (17) of the fluidic component (8), and a connector window (27) for receiving the spring-loaded contacts (16).

9. System according to claims 1 to 8, further comprising magnets arranged for trapping magnetic nanoparticles in the microfluidic channels (9).

10. System according to any of the preceding claims, comprising instrumentation to carry out the electrochemical detection by chronoamperometry of the multiplexed biosensor.

## Patentansprüche

1. Biosensorsystem zur multiplexen Detektion von Biomarkern, wobei das System Folgendes umfasst:
eine Reihe von mindestens zwei elektrochemischen Zellen (1a, 1b, 1c, 1d, 1e), welche dazu angepasst sind, einzeln adressierbar zu sein,
eine Arbeitselektrode (2a, 2b, 2c, 2d, 2e) in jeder elektrochemischen Zelle (1a, 1b, 1c, 1d, 1e) der Reihe,
eine fluidische Komponente (8) hergestellt aus einem porösen Material zum Treiben von Fluiden mittels Kapillarwirkung zu den elektrochemischen Zellen (1a, 1b, 1c, 1d, 1e) hin,
eine Kartusche (19), welche dafür ausgebildet ist, die Reihe und die fluidische Komponente (8) für die elektrochemische Detektion von biologischen Proben auszurichten und in Kontakt zu bringen;
eine geteilte Gegenelektrode (6) und eine geteilte Referenzelektrode (7), und
ein Chipsubstrat (30), welches innerhalb der Kartusche (19) eingeschlossen ist,
wobei die fluidische Komponente (8) aus Papier aufweisend mikrofluidische Kanäle (9), beinhaltend Biokomponenten, hergestellt ist, wobei die mikrofluidischen Kanäle (9) voneinander isoliert sind, wobei die fluidische Komponente (8) mit der Reihe von elektrochemischen Zellen (1a, 1b, 1c, 1d, 1e) betriebsfähig koppelbar ist, sodass jeder mikrofluidische Kanal (9) über einer elektrochemischen Zelle platziert wird, um die elektrochemische Detektion vorzunehmen;
wobei die geteilte Gegenelektrode und die geteilte Referenzelektrode (6, 7) gemeinsame Elektroden für alle elektrochemischen Zellen (1a, 1b, 1c, 1d, 1e) der Reihe sind;
wobei die Gegenelektrode (6), die Referenzelektrode (7) und die Arbeitselektroden (2a, 2b, 2c, 2d, 2e) auf der gleichen Seite des Chipsubstrats (30) gebildet sind; und
wobei die mikrofluidischen Kanäle (9) mit Antikörpern oder DNA-Strängen versehen sind, um Affinitätstests vorzunehmen.

2. System nach Anspruch 1, wobei die geteilte Gegenelektrode (6) und die geteilte Referenzelektrode (7) gerade Bahnen sind, welche parallel zueinander und parallel zu einer Längsausrichtungsrichtung der Arbeitselektroden (2a, 2b, 2c, 2d, 2e) sind, und wobei die Arbeitselektroden (2a, 2b, 2c, 2d, 2e) ausgerichtet und zwischen der geteilten Gegenelektrode und einer geteilten Referenzelektrode angeordnet sind.

3. System nach einem der vorhergehenden Ansprüche, zusätzlich umfassend einen Satz von Anschlusspolstern (3), und einen Satz von Anschlussbahnen (4), welche die Arbeitselektroden (2a, 2b, 2c, 2d, 2e), die geteilte Gegenelektrode und die geteilte Referenzelektrode (6, 7) mit den Anschlusspolstern (3) anschließen, wobei ein Teil jeder Anschlussbahn (4) parallel zur geteilten Gegenelektrode und zur geteilten Referenzelektrode ist.

4. System nach Anspruch 1, wobei das Chipsubstrat (30) rechteckig ist und die Anschlusspolster (3) in einer netzartigen Matrixanordnung gruppiert sind und einer kurzen Seite des Substrats benachbart gebildet sind, und wobei das System zusätzlich federbelastete Kontakte (16) umfasst, welche an die Anschlusspolster (3) angeschlossen sind.

5. System nach einem der Ansprüche 1 bis 4, wobei die mikrofluidischen Kanäle (9) die Form von geraden Streifen aufweisen, sodass, wenn die fluidische Komponente (8) mit den elektrochemischen Zellen (1a, 1b, 1c, 1d, 1e) gekoppelt ist, die mikrofluidischen Kanäle (9) quer in Bezug auf die geteilte Gegenelektrode und die geteilte Referenzelektrode (6, 7) angeordnet sind.

6. System nach einem der vorhergehenden Ansprüche, wobei die Kartusche (19) obere und untere Teile (20, 21) und Klemmstrukturen (22, 23, 24, 25), welche dazu angepasst sind, die oberen und unteren Teile (20, 21) der Kartusche (19) zu befestigen und zu drücken, um die Reihe und die fluidische Komponente in engem und stabilem Kontakt zu halten, aufweist.

7. System nach einem der Ansprüche 1 bis 6, zusätzlich umfassend ein Absorptionspolster (11) in Kontakt mit den mikrofluidischen Kanäle (9) der fluidischen Komponente (8) und denselben teilweise überlappend.

8. System nach Anspruch 7, wobei das obere Teil (20) der Kartusche (19) ein Verdampfungsfenster (20a), welches über dem Absorptionspolster (11) platziert ist und als Senkbereich-Verdampfungsfenster dient, und ein Probenzugangsfenster (26), welches direkt über einem ersten Fenster (17) der fluidischen Komponente (8) platziert ist, und ein Anschlussfenster (27) zum Aufnehmen der federbelasteten Kontakte (16), aufweist.

9. System nach den Ansprüchen 1 bis 8, zusätzlich umfassend Magnete, welche dafür angeordnet sind, magnetische Nanopartikel in den mikrofluidischen Kanälen (9) zu fangen.

10. System nach einem der vorhergehenden Ansprüche, umfassend eine Geräteausrüstung, um die elektrochemische Detektion mittels Chronoamperometrie des Multiplexbiosensors vorzunehmen.

## Revendications

1. Système de biocapteur pour la détection de biomarqueurs multiplexée, le système comprenant :
un réseau d'au moins deux cellules électrochimiques (1a, 1b, 1c, 1d, 1e), adaptées pour être individuellement adressées,
une électrode de travail (2a, 2b, 2c, 2d, 2e) dans chaque cellule électrochimique (1a, 1b, 1c, 1d, 1e) du réseau,
un composant fluidique (8) réalisé en un matériau poreux pour conduire des fluides par action capillaire vers les cellules électrochimiques (1a, 1b, 1c, 1d, 1e),
une cartouche (19) configurée pour aligner et mettre en contact le réseau et le composant fluidique (8) pour la détection électrochimique d'échantillons biologiques ;
une contre-électrode partagée (6) et une électrode de référence partagée (7), et
un substrat de puce (30) enfermé à l'intérieur de la cartouche (19),
dans lequel le composant fluidique (8) est réalisé en papier ayant des canaux microfluidiques (9) comportant des composants biologiques, dans lequel les canaux microfluidiques (9) sont isolés les uns des autres, dans lequel le composant fluidique (8) peut être couplé fonctionnellement avec le réseau de cellules électrochimiques (1a, 1b, 1c, 1d, 1e) de telle sorte que chaque canal microfluidique (9) est placé sur une cellule électrochimique pour mettre en œuvre la détection électrochimique ;
dans lequel la contre-électrode et l'électrode de référence partagées (6, 7) sont des électrodes communes pour toutes les cellules électrochimiques (1a, 1b, 1c, 1d, 1e) du réseau ;
dans lequel la contre-électrode (6), l'électrode de référence (7), et les électrodes de travail (2a, 2b, 2c, 2d, 2e) sont formées sur le même côté du substrat de puce (30) ; et
dans lequel les canaux microfluidiques (9) sont pourvus d'anticorps ou de brins d'ADN pour mettre en œuvre des tests d'affinité.

2. Système selon la revendication 1, dans lequel la contre-électrode partagée (6) et l'électrode de référence partagée (7) sont des pistes droites parallèles les unes aux autres, et parallèles à une direction d'alignement longitudinale des électrodes de travail (2a, 2b, 2c, 2d, 2e), et dans lequel les électrodes de travail (2a, 2b, 2c, 2d, 2e) sont alignées et disposées entre la contre-électrode partagée et une électrode de référence partagée.

3. Système selon l'une quelconque des revendications précédentes, comprenant en outre un ensemble de patins de connexion (3), et un ensemble de pistes de connexion (4) reliant les électrodes de travail (2a, 2b, 2c, 2d, 2e), le contre-électrode partagée et l'électrode de référence partagée (6, 7), avec les patins de connexion (3), dans lequel une partie de chaque piste de connexion (4) est parallèle à la contre-électrode partagée et l'électrode de référence partagée.

4. Système selon la revendication 1, dans lequel le substrat de puce (30) est rectangulaire et les patins de connexion (3) sont grouppées dans une disposition de matrice réticulaire et formées adjacentes à un petit côté du substrat, et dans lequel le système comprend en outre des contacts chargés par ressort (16) reliés avec les patins de connexion (3).

5. Système selon l'une quelconque des revendications 1 à 4, dans lequel les canaux microfluidiques (9) présentent la forme de bandes droites, de telle sorte que, lorsque le composant fluidique (8) est couplé avec les cellules électrochimiques (1a, 1b, 1c, 1d, 1e), les canaux microfluidiques (9) sont disposés transversalement par rapport à la contre-électrode et l'électrode de référence partagées (6, 7).

6. Système selon l'une quelconque des revendications précédentes, dans lequel la cartouche (19) présente des parties supérieure et inférieure (20, 21) et des structures de serrage (22, 23, 24, 25) adaptées pour attacher et presser les parties supérieure et inférieure (20, 21) de la cartouche (19) pour maintenir le réseau et le composant fluidique en contact étroit et stable.

7. Système selon l'une quelconque des revendications 1 à 6, comprenant en outre un coussin absorbent (11) en contact et chevauchant partiellement les canaux microfluidiques (9) du composant fluidique (8).

8. Système selon la revendication 7, dans lequel la partie supérieure (20) de la cartouche (19) présente une fenêtre d'évaporation (20a) qui est placée sur le coussin absorbent (11), et sert de fenêtre d'évaporation de zone de dissipation, et une fenêtre d'accès à l'échantillon (26) qui est placée directement au-dessus d'une première fenêtre (17) du composant fluidique (8), et une fenêtre de connecteur (27) pour recevoir les contacts chargés par ressort (16).

9. Système selon les revendications 1 à 8, comprenant en outre des aimants disposés pour piéger des nanoparticules magnétiques dans les canaux microfluidiques (9).

10. Système selon l'une quelconque des revendications précédentes, comprenant des instruments pour mettre en œuvre la détection électrochimique par chronoampérométrie du biocapteur multiplexé.
